(19) Europäisches Patentamt
European Patent Office
Office européen des brevets

(11) Publication number: **0 003 884**
**A2**

(12) # EUROPEAN PATENT APPLICATION

(21) Application number: **79300216.3**

(22) Date of filing: **13.02.79**

(51) Int. Cl.²: **A 01 N 9/22**
A 01 N 9/12, C 07 D 233/60
C 07 D 249/08

(30) Priority: **23.02.78 GB 732378**

(43) Date of publication of application:
**05.09.79 Bulletin 79/18**

(84) Designated contracting states:
**BE CH DE FR GB IT NL SE**

(71) Applicant: **IMPERIAL CHEMICAL INDUSTRIES LIMITED**
**Imperial Chemical House Millbank**
**London SW1P 3JF(GB)**

(72) Inventor: **Lewis, Terence**
**18 Quintilis Roman Hill**
**Bracknell Berkshire(GB)**

(72) Inventor: **Worthington, Paul Anthony**
**22 Boulters Gardens**
**Maidenhead Berkshire(GB)**

(74) Representative: **Downes, John Edward et al,**
**Imperial Chemical Industries Limited Legal Department: Patents Thames House North Millbank**
**London SW1P 4QG(GB)**

(54) **Alpha-beta unsaturated ketone derivatives useful as herbicides.**

(57) Herbicidal compounds of the formula (I):

$$R^1-\underset{\underset{A}{\|}}{C}(H)C=C(X)R^2 \qquad (I)$$

wherein A is oxygen or sulphur; X is a 1-(1,2,4- triazolyl) or 1-imidazolyl radical; $R^1$ is an aliphatic or cycloaliphatic radical substituted by one or more of the following:
halogen, hydroxy, alkoxy, acyloxy, alkoxycarbonyl, cyano, carboxamido, nitro, mono- or di-alkyl carbamoyl, acylamido, mono- or di-alkylamino, phenyl, or mono- or di-alkylsulphonamido; and $R^2$ is a phenyl or naphthyl radical optionally bearing one or more of a group of specified substituents, including halogen, alkyl, and alkoxy; and acid addition salts and metal complexes of the compounds (I).

EP 0 003 884 A2

Croydon Printing Company Ltd.

30060/EP



# ALPHA-BETA UNSATURATED KETONE DERIVATIVES USEFUL AS HERBICIDES

This invention relates to chemical compounds having herbicidal properties, and to herbicidal processes and compositions utilising these compounds.

According to the present invention, there are provided herbicidal compounds having the formula (I):-

$$R^1-\underset{\underset{A}{\|}}{C}(H)C=C(X)R^2$$

(I)

wherein A is an atom of oxygen or sulphur; X is a 1-(1,2,4-triazolyl) radical or a 1-imidazolyl radical; and $R^1$ is an aliphatic or cycloaliphatic radical substituted by one or more of the following substituents: fluorine; chlorine; bromine; iodine; alkoxy; hydroxy; alkoxycarbonyl; acyloxy; cyano; carboxamido; nitro; mono- or di-alkylcarbamoyl; acylamido; mono- or dialkyl amino; phenyl; or mono- or dialkylsulphonamido; and $R^2$ is a phenyl or naphthyl radical optionally bearing one or more of the following substituents: fluorine; chlorine; bromine; iodine; cyano; nitro; alkyl of 1 to 6 carbon atoms optionally substituted by one or more alkoxy radicals or fluorine, chlorine, bromine, or iodine atoms; hydroxy;

alkoxy of 1 to 6 carbon atoms optionally substituted by one or more phenyl radicals or alkoxy radicals of 1 to 6 carbon atoms; alkenyloxy or alkynyloxy of up to 6 carbon atoms; methylenedioxy or ethylenedioxy radicals optionally bearing one or more alkyl substituents in the methylene or ethylene moiety; alkylthio of 1 to 6 carbon atoms; phenyl; phenoxy; amino; mono- or di- alkylamino; acylamino; carboxy; carbamoyl; mono- or di- alkylcarbamoyl; sulphamoyl; or mono-or di- alkylsulphamoyl; or an acid addition salt or metal complex thereof.

One sub-group of compounds within the above definition comprises compounds of formula (I) in which $R^2$, A and X are as hereinbefore defined, and $R^1$ is a group

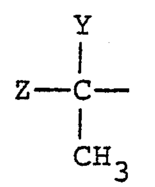

wherein Y is a methyl group or a 2-cyanoethyl group, and Z is a hydroxy group; an alkoxy group of 1 to 6 carbon atoms; an alkanoyloxy group of 2 to 6 carbon atoms; a benzyloxy group optionally substituted by one or more methyl group or atoms of fluorine, chlorine, bromine or iodine; or a 2-cyanoethyl group.

Another sub-group of compounds according to the invention comprises compounds in which $R^1$ is a

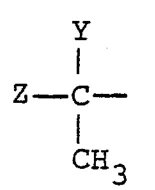

group as hereinbefore defined, A is oxygen and $R^2$ is a phenyl or naphthyl radical optionally substituted by one or more alkyl groups of 1 to 4 carbon atoms, alkoxy groups of 1 to 6 carbon atoms, or fluorine, chlorine, bromine, or iodine atoms. When $R^2$ is a substituted phenyl group it may be for example a 2,5 or 2,6-disubstituted phenyl group. Examples of 2,5-disubstituted phenyl groups include 2-alkoxy-5-halogeno phenyl groups, for example 2-methoxy-5-bromophenyl.

The identity of the acid which is used to form the acid addition salts of the compounds of the invention is not critical and a wide variety of acid addition salts of any particular compound may therefore be used. For reasons of convenience and economy, however, salts derived from the readily available mineral acids are preferred, although others may be used if desired. In considering the choice of acid, the purpose for which the salt is to be used will be taken into account; salts formed from herbicidal acids which are highly persistent in soil would obviously not be suitable for applications in which crops are to be planted shortly after the herbicide is applied. Particular examples of acids which may be used to form the acid addition salts include hydrochloric, hydrobromic, sulphuric, nitric, phosphoric, and *p*-toluenesulphonic acids.

The compounds of the invention form complexes with salts of transition metals, for example copper and zinc salts, and such complexes form part of the invention. Conveniently the transition metal salts are halides, sulphates or nitrates. The metal complexes usually contain two molar proportions of the compounds of the invention to one molar proportion of the metal salt. They may be prepared by mixing solutions of the compound and the metal salt and collecting the precipitated complex. The solvent may be, for example, a lower alkanol, for example methanol or ethanol.

Subgroups of compounds falling within the general class of compounds defined above include the group in which $R^1$ is a substituted alkyl or cycloalkyl radical. When $R^1$ is a substituted alkyl radical it may be for example a branched chain radical.

The compounds of the invention are capable of existing in two geometrically isomeric forms (*cis* and

trans), according to the disposition of the groups attached to the carbon atoms linked by a double bond. Both isomers, and mixtures thereof, form part of the invention. Generally in a given preparation one isomer is produced in greater proportion than the other. If desired, the isomers can be separated by conventional chemical methods, for example by gas-liquid chromatography (GLC). It may be more convenient, however, to use the mixture of isomers as a herbicide. The proportion of each isomer in the mixture may readily be determined by physical methods of analysis. for example by examination of the nuclear magnetic resonance spectrum of the product since the spectra of the two isomers differ from each other. The two isomers of a given compound may not be completely identical in biological activity in every case.

Particular example of compounds according to the invention are listed in Table I below.

TABLE I

| COMPOUND NO | $R^1$ | $R^2$ | A | X | MELTING POINT °C |
|---|---|---|---|---|---|
| 1 | $CH_3OC(CH_3)_2-$ | $.C_6H_4.OCH_3$<u>o</u> | O | T | 56-70 |
| 2 | $CH_3OC(CH_3)_2-$ | $.C_6H_4.OCH_3$<u>o</u> | O | I | 86-94 |
| 3 | $CH_3COOC(CH_3)_2-$ | $.C_6H_4.OCH_3$<u>o</u> | O | T | 121 |
| 4 | $(CNCH_2CH_2)_2C(CH_3)-$ | $.C_6H_4.OCH_3$<u>o</u> | O | T | oil |
| 5 | $HOC(CH_3)_2-$ | $.C_6H_4.Cl$<u>p</u> | O | T | 110-112 |
| 6 | $CH_3OC(CH_3)_2-$ | $2-CH_3O-5-Br-C_6H_3.$ | O | T | 125-126 |
| 7 | $CH_3OC(CH_3)_2-$ | $2-CH_3O-5-Br-C_6H_3.$ | O | I | 120 |
| 8 | $CH_3OC(CH_3)_2-$ | $2,5-(CH_3)_2-C_6H_3.$ | O | T | 81-82 |
| 9 | $CH_3OC(CH_3)_2-$ | 1-naphthyl | O | I | oil |
| 10 | $CH_3OC(CH_3)_2-$ | 1-naphthyl | O | T | oil |
| 11 | $CH_3OC(CH_3)_2-$ | $2,6-(CH_3)_2-C_6H_3.$ | O | T | 89-90 |
| 12 | $CH_3OC(CH_3)_2-$ | $2,6-(CH_3)_2-C_6H_3.$ | O | I | oil |
| 13 | $C_2H_5OC(CH_3)_2-$ | $2-CH_3O-C_6H_4$ | O | I | oil |
| 14 | $CH_3OC(CH_3)_2-$ | $2,6-Cl_2-C_6H_3.$ | O | T | 154-156 |
| 15 | $CH_3OC(CH_3)_2-$ | $2,6-Cl_2-C_6H_3.$ | O | I | 101-102 |
| 16 | $CH_3OC(CH_3)_2-$ | $2,6-(CH_3O)_2-C_6H_3.$ | O | T | 110-112 |
| 17 | $CH_3OC(CH_3)_2-$ | $2,6-(CH_3O)_2-C_6H_3.$ | O | I | 119-120 |
| 18 | $HOC(CH_3)_2-$ | $2,4-Cl_2.C_6H_3$ | O | T | oil |
| 19 | $HOC(CH)_2-$ | $2-Cl.C_6H_4$ | O | T | 119-120 |
| 20 | $C_2H_5OC(CH_3)_2-$ | $2-CH_3O-5-Br-C_6H_3.$ | O | T | 89-90 |
| 21 | $C_2H_5OC(=O)C(CH_3)_2.$ | $2-CH_3OC_6H_4.$ | O | T | oil |

Further examples of compounds falling within the scope of the invention are listed in Table II below.

TABLE II

| $R^1$ | $R^2$ | A | X |
|---|---|---|---|
| $C_6H_5O\underset{\underset{O}{\|}}{C}C(CH_3)_2-$ | $2-CH_3O.C_6H_4.$ | O | T |
| $C_2H_5OC(CH_3)_2-$ | $2-CH_3O-5-Br-C_6H_3.$ | O | I |
| $C_2H_5OC(CH_3)_2-$ | $2,6-(CH_3)_2-C_6H_3.$ | O | T |
| $C_2H_5OC(CH_3)_2-$ | $2,6-(CH_3)_2-C_6H_3.$ | O | I |
| $CH_3OC(CH_3)_2-$ | $2-C_6H_{13}O.C_6H_4.$ | O | I |
| $CH_3OC(CH_3)_2-$ | $2-C_6H_{13}O.C_6H_4.$ | O | T |
| $C_4H_9OC(CH_3)_2-$ | $2-CH_3O.C_6H_4.$ | O | T |
| $CNCH_2CH_2C(CH_3)_2-$ | $2-CH_3O.C_6H_4.$ | O | T |
| $CH_3OCOCH_2CH_2C(CH_3)_2$ | $2-CH_3O.C_6H_4.$ | O | T |
| $(CH_3)_2CH\underset{\underset{O}{\|}}{C}OC(CH_3)_2-$ | $2-CH_3O.C_6H_4.$ | O | T |

In the foregoing tables, the symbol I stands for a 1-imidazolyl radical and T stands for a 1-(1,2,4-triazolyl) radical.

In another aspect the invention provides a process of killing or severely injuring unwanted plants, which comprises applying to the plants, or to the growth medium thereof, a compound of the formula (I):-

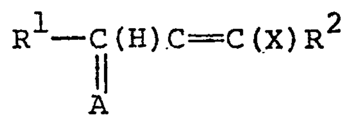

(I)

wherein $R^1$, $R^2$, A and X are as hereinbefore defined, or an acid addition salt or metal complex thereof.

As will be understood by those skilled in the art, the amount of the compound (I) to be applied will depend upon a variety of factors, for example the particular compound chosen for use and the identity of the unwanted plants. By way of general guidance, however, a rate of from 0.01 to 10 kilograms per hectare is usually suitable, while from 0.25 to 5 kilograms per hectare is usually preferred.

The compounds of the invention are in general broad-spectrum herbicides, that is to say, they are toxic towards a wide variety of plant species. A valuable feature of the invention is that the compounds are often effective in the control of the weed Cyperus rotundus, a species which is difficult to control with known herbicides.

Although the compounds (I) are in general broad spectrum herbicides, they include compounds which are relatively less toxic towards certain crop species and which may therefore be used as selective herbicides in these crops. Examples of such compounds include compounds 7 and 11 which at low rates of application show relatively little damage towards cotton, soya, and maize, while causing substantial damage to a number of weed species.

The compounds used in the process of the invention are preferably applied in the form of a composition, in which the active ingredient is mixed with a diluent or carrier. In another aspect, therefore, the invention provides a herbicidal composition, comprising as an active ingredient a compound of formula (I) as hereinbefore defined, in admixture with a solid or liquid diluent. Preferably the composition also comprises a surface-active agent.

The solid compositions of the invention may be for example, in the form of dusting powders, or may take the form of granules. Suitable solid diluents include, for example, kaolin, bentonite, kieselguhr, dolomite, calcium carbonate, talc, powdered magnesia, and Fuller's earth.

Solid compositions may also be in the form of dispersible powders or grains comprising in addition to the active ingredient, a wetting agent to facilitate the dispersion of the powder or grains in liquids. Such powders or grains may include fillers, suspending agents and the like.

Liquid compositions include aqueous solutions, dispersions and emulsions containing the active ingredient preferably in the presence of one or more surface-active agents. Water or organic liquids may be used to prepare solutions, dispersions, or emulsions of the active ingredient. The liquid compositions of the invention may also contain one or more corrosion inhibitors for example lauryl isoquinolinium bromide.

Surface-active agents may be of the cationic, anionic or non-ionic type. Suitable agents of the cationic type include for example quaternary ammonium compounds, for example cetyltrimethyl ammonium bromide. Suitable agents of the anionic type include for example soaps, salts of aliphatic mono-esters of sulphuric acid, for example sodium lauryl sulphate; and salts of sulphonated aromatic compounds, for example dodecylbenzenesulphonate, sodium, calcium and ammonium lignosulphonate, butylnaphthalene

sulphonate, and a mixture of the sodium salts of diisopropyl- and triisopropyl-naphthalenesulphonic acid. Suitable agents of the non-ionic type include, for example, the condensation products of ethylene oxide with fatty alcohols such as oleyl alcohol and cetyl alcohol, or with alkyl phenols such as octyl-phenol, nonylphenol, and octylcresol. Other non-ionic agents are the partial esters derived from long chain fatty acids and hexitol anhydrides, for example sorbitol mono-laurate; the condensation products of the said partial esters with ethylene oxide and the lecithins.

The compositions which are to be used in the form of aqueous solutions, dispersions or emulsions are generally supplied in the form of a concentrate containing a high proportion of the active ingredient, the concentrate being diluted with water before use. These concentrates are usually required to withstand storage for prolonged periods and after such storage to be capable of dilution with water in order to form aqueous preparations which remain homogeneous for a sufficient time to enable them to be applied by conventional spray equipment. In general concentrates may conveniently contain from 10 to 85% and preferably from 25 to 60% by weight of active ingredient. Dilute preparations ready for use may contain varying amounts of the active ingredient, depending upon the purpose for which they are to be used; however, dilute preparations suitable for many uses contain between 0.01% and 10% and preferably between 0.1% and 1% by weight of the active ingredient.

In another aspect, the invention provides processes for preparing compounds of the formula:-

$$R^1-\underset{\underset{A}{\|}}{C}(H)C=C(X)R^2$$

(I)

wherein $R^1$, $R^2$, A and X have the meanings previously assigned to them in this specification.

A method of preparing compounds of formula (I) is outlined in Scheme A.

Scheme A

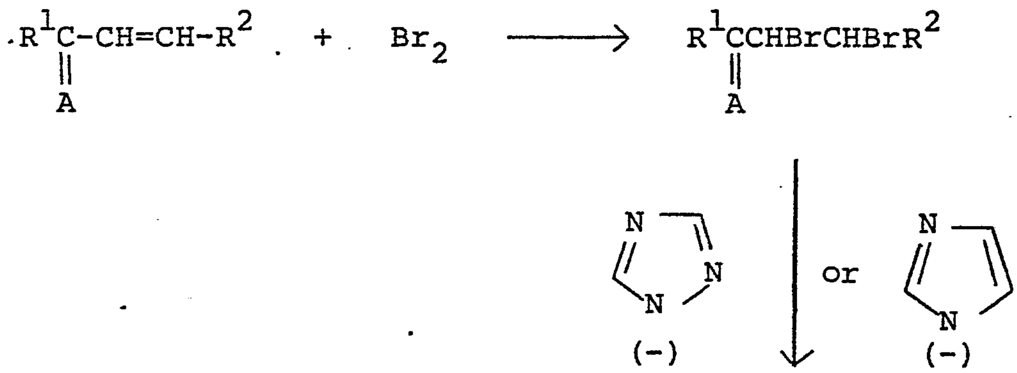
or

(I)

In Scheme A, a suitably substituted ketone (II) is treated with bromine to give the dibromo compound (III). This dibromo compound is then reacted with the anion of 1,2,4-triazole or imidazole to give the required compound (I). The anion of 1,2,4-triazole or imidazole is obtained by treating the triazole or imidazole with a base in a suitable solvent. Examples of suitable solvents include aprotic solvents, for example hydrocarbons and ethers, and in particular dimethylformamide. Conveniently the base employed to form the anion is an alkali metal hydride, for example sodium hydride. The formation of the anion by treatment with base is preferably carried out at room temperature or below. The reaction mixture containing the anion may conveniently be used directly in the subsequent reaction. The subsequent reaction with the α,β-dihalogeno ketone is preferably carried out at a higher temperature, for example at 100 to 120°C. The product may be isolated by conventional methods, for example by pouring the reaction mixture into water, extracting with an organic solvent, evaporating the

extracts, and purifying the residue by conventional methods, for example by recrystallisation. The solvent for the reaction with the dihalogenoketone may conveniently be the one in which the anion was prepared.

The unsaturated ketones required as starting materials are known compounds or may be prepared by processes known in themselves.

Compounds in which A represents sulphur may be obtained by reacting compounds in which A is oxygen with phosphorus pentasulphide, according to known procedures.

In a further aspect the present invention provides compounds of formula (I) whenever prepared by the processes herein described.

If desired, the compounds of the invention may be applied in admixture with other herbicides to inhibit the growth of unwanted plants.

The invention is illustrated by the following Examples, in which all parts are by weight and all temperatures in degrees Centigrade unless otherwise stated. Examples 1 to 5 illustrate the preparation of compounds 1 to 5 of Table I. The remaining compounds in Table I were prepared by methods analogous to those described in Examples 1 to 5.

EXAMPLE 1

This Example illustrates the preparation of 1(2-methoxyphenyl)-1-(1,2,4-triazol-1-yl)-4-methoxy-4-methylpent-1-en-3-one (Compound no 1 of Table I).

(a) Preparation of 1-(2-methoxyphenyl)-4-methoxy-4-methyl-pent-1-en-3-one.

2-Methyl-2-methoxybutan-3-one (12.76 g) and o-anisaldehyde (13.6 g) were added to a solution of sodium methoxide in methanol prepared by adding sodium (2.53 g) to methanol (75 ml). The mixture was stirred and heated under reflux for 1.5 hours.

The mixture was then diluted with water (75 ml) and extracted twice with ether. The ether extracts were washed three times with water, dried, and evaporated.

The light red oil which remained was distilled at 136-142°C/ 0.05 Torr to obtain the product.

(b) Preparation of compound no 1.

The product from (a) (15.0 g) in carbon tetrachloride (50 ml) was treated dropwise with bromine (4 ml) in carbon tetrachloride (10 ml) at a temperature between -5 to +5°C. The solution was then evaporated. The remaining oil rapidly solidified and was recrystallised from hexane to give the dibromo compound, melting point 108-110°C.

A solution of this dibromo compound (10.0 g) in dimethylformamide (15 ml) was added to a solution prepared by adding a solution of 1,2,4-triazole (5.2 g) in dimethylformamide (15 ml) dropwise with stirring to a suspension of sodium hydride (3.6 g of 50% dispersion in mineral oil, washed free of oil with hexane) while cooling in cold water. The mixture was then heated at 100°C for 1 hour, poured into water, and extracted twice with ether. The ether extracts were then washed three times with water, dried, and evaporated to leave a yellow oil. This was purified on a column of silica gel using diisopropyl ether as the eluent. The product (compound no 1) had a melting range of 56-70°C.

EXAMPLE 2

This Example illustrates the preparation of 1-(1-imidazolyl)-1-(2-methoxyphenyl)-4-methoxy-4-methyl-pent-1-en-3-one.

A solution of imidazole (2.8 g) in dry dimethylformamide (7 ml) was added dropwise to a suspension of sodium hydride (1.92 g of 50% dispersion in mineral oil, washed free of oil with hexane) with stirring and cooling. The dibromo compound from paragraph (b) of Example 1 above (5.4 g) in dimethylformamide (7 ml) was then added and the mixture heated for 1 hour at 100°C and then poured into water. The mixture was extracted twice with ether and the ether extracts washed three times with

water, dried, and evaporated. The yellow oil remaining was purified by passage through a column of silica gel, using diisopropyl ether as the eluent. The product (compound no 2) had a melting point of 86-94°C.

EXAMPLE 3

This Example illustrates the preparation of Compound no 3 of Table I.

(a) Preparation of 4-acetoxy-1-(2-methoxyphenyl)-4-methyl-pent-1-en-3-one.

A mixture of 1-(2-methoxyphenyl)-4-hydroxy-4-methyl-pent-1-en-3-one (5 g), sodium acetate (2.5 g) and acetic anhydride (7.5 ml) was heated under reflux for 1 hour. The solvent was removed and the residue taken up in a small volume of ethanol. On standing, yellow crystals of the acetoxy compound separated and were recrystallised from petroleum ether (b.p. 40-60°C).

(b) Preparation of 4-acetoxy-1,2-dibromo-1-(2-methoxyphenyl)pentan-3-one.

The product from (a) (2.6 g) was dissolved in carbon tetrachloride (50 ml) and the solution cooled to -10°C. Bromine (1.69 g) in carbon tetrachloride (20 ml) was added portionwise keeping the temperature at or below -10°C. The solution was then warmed to room temperature and evaporated to give the dibromo compound as a colourless oil.

(c) Preparation of compound no 3

1,2,4-Triazole (1.93 g) in dry dimethylformamide (20 ml) was added dropwise to sodium hydride (50% suspension in mineral oil; 1.40 grams washed free of oil with hexane). The suspension was stirred for 20 minutes at room temperature and cooled to -20°C while a solution of the dibromo compound (3.90 g) from paragraph (b) in dry dimethylformamide was added. After the initial effervescence subsided the orange solution was heated on a steam bath for 30 minutes. The mixture was then poured into

water and the oil which separated extracted with ether. The extracts were washed with water, dried, and evaporated. The residue was purified by preparative thin layer chromatography to give 4-acetoxy-1-(2-methoxyphenyl)-4-methyl-1-(1,2,4-triazol-1-yl)pent-1-en-3-one (compound no 3 of Table I) as colourless needles with a melting point of 121°C.

EXAMPLE 4

This Example illustrates the preparation of 6-cyano-4-(2-cyanoethyl)-1-(2-methoxyphenyl)-4-methyl-1-(1,2,4-triazol-1-yl)hex-1-en-3-one (compound no 4 of Table I).

(a) Preparation of 6-cyano-4-(2-cyanoethyl)-1-(2-methoxyphenyl)-4-methyl-hex-1-en-3-one.

A suspension of 5-cyano-3-(2-cyanoethyl)3-methylpentan-2-one (17.8 g) and 2-methoxy benzaldehyde (15 g) in ethanol (100 ml) was added portionwise to a solution of potassium hydroxide (5.6 g) in water (100 ml) with stirring. The mixture was stirred for 2.5 hours and the solid filtered off, washed with water, and recrystallised from ethanol to give the hex-1-en-3-one derivative as needles with a melting point of 126°C.

(b) Preparation of 6-cyano-4(2-cyanoethyl)-1,2-dibromo-1-(2-methoxyphenyl)-4-methylhexan-3-one.

The product from (a) above (20 g) in chloroform (100 ml) was cooled to -5°C or below while bromine (10.85 g) was added dropwise. The solution was stirred at room temperature for 15 minutes and evaporated. The residue was recrystallised from hexane to give the dibromo compound as colourless needles of melting point 138°C.

(c) Preparation of compound no 4 of Table I

1,2,4-Triazole (3.63 g) in dry dimethylformamide (10 ml) added dropwisee to a suspension of sodium hydride (2.52 g of 50% sodium hydride in mineral oil, washed free of oil with hexane) in dimethylformamide (10 ml). The suspension was stirred for 20 minutes and

cooled to -20°C or below while a solution of the dibromo compound (8 g) from (b) above in dimethylformamide (20 ml) was added in portions. The solution was heated at 100°C for 1 hour, and then poured into water (200 ml). The oil which separated was extracted with chloroform. The chloroform extracts were washed with water, dried, and evaporated to yield an orange oil. The oil was taken up in toluene (20 ml) and the solution added to a solution of p-toluenesulphonic acid (2.3 g) in toluene (20 ml) and ethanol (5 ml). The mixture was heated on the steambath for 30 minutes, cooled, and diluted with hexane. The oil which separated was separated by decantation and stirred with water (50 ml), ethanol (5 ml) and concentrated ammonia solution (5 drops) for 30 minutes. The mixture was extracted with chloroform and the extracts washed with water, dried, and evaporated to yield compound no 4 as an orange oil.

EXAMPLE 5

This Example describes the preparation of 1-(1,2,4-triazol-1-yl)-1-(p-chlorophenyl)-4-hydroxy-4-methyl-pent-1-en-3-one (compound no 5).

(a) Preparation of 1-phenyl-4-hydroxy-4-methyl-pent-1-en-3-one

p-Chlorobenzaldehyde (28.1 g) and 3-hydroxy-3-methylbutan-2-one (20.4 g) were mixed together at 0°C. Powdered potassium hydroxide (11.2 g) was added in portions at 0°C. The mixture was stirred at 0°C for 1 hour. Water (100 ml) was added. The oil which separated solidified and was washed with cold petroleum (2 x 100 ml) and dried. Recrystallisation from petroleum/chloroform gave the pent-1-en-3-one derivative with a melting point of 59-60°C.

(b) Preparation of 1-phenyl-1,2-dibromo-4-hydroxy-4-methylpentan-3-one.

The product from (a) (9.0 g) and pyridinium

hydrobromide perbromide (14.1 g) were stirred in acetic acid (100 ml) at 25°C for 2 hours, until the red colouration had disappeared. Water (100 ml) was added and the colourless solid which separated was collected, washed with water, and dried. Recrystallisation from petroleum/chloroform gave the dibromo compound with a melting point of 117-118°C.

(c) Preparation of compound no 5

Sodium hydride (0.72 g) was suspended in dry dimethylformamide (50 ml). 1,2,4-Triazole (2.1 g) was added in portions and the mixture stirred until the triazole had dissolved. The product from (b) (5.76 g) was added in portions with cooling to keep the temperature at 25°C. After stirring for 24 hours at room temperature the solution was poured into water (100 ml). The mixture was extracted with chloroform (100 ml), and the extract washed with water (3 x 100 ml), dried, and evaporated to give a yellow oil which was purified by passage through a column of silica gel, using a mixture of equal volumes of petroleum and ethyl acetate as the solvent. The product (compound no 5) was obtained as a yellow oil which slowly crystallised to a solid with a melting point of 110-112°C.

EXAMPLE 6

This Example illustrates the herbicidal properties of the compounds of the invention. Each compound was formulated for test by mixing an appropriate amount with 5 ml of an emulsion prepared by diluting 160 ml of a solution containing 21.8 grams per litre of Span 80 and 78.2 grams per litre of Tween 20 in methyl cyclohexanone to 500 ml with water. Span 80 is a Trade Mark for a surface-active agent comprising sorbitan mono-laurate. Tween 20 is a Trade Mark for a surface-active agent comprising a condensate of twenty molar proportions of ethylene oxide with sorbitan mono-oleate. The mixture of

the compound and the emulsion was shaken with glass beads and diluted to 40 ml with water.

The spray composition so prepared was sprayed on to young pot plants (post-emergence test) of the species named in Table III below, at a rate equivalent to 1000 litres per hectare. Damage to plants was assessed 14 days after spraying by comparison with untreated plants, on a scale of 0 to 5 where 0 is 0 to 20% damage and 5 represents 100% kill. In a test for pre-emergence herbicidal activity, seeds of the test species were placed on the surface of fibre trays of soil and were sprayed with the compositions at the rate of 1000 litres per hectare. The seeds were then covered with further soil. Three weeks after spraying, the seedlings in the sprayed fibre trays were compared with the seedlings in unsprayed control trays, the damage being assessed on the same scale of 0 to 5. The results are given in Table III below:-

TABLE III

| COMPOUND NO | A | B | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
|---|---|---|---|---|---|---|---|---|---|---|---|---|---|---|---|---|---|---|---|---|---|---|---|---|---|---|
| | | | TEST PLANTS | | | | | | | | | | | | | | | | | | | | | | | |
| 4 | 1.0 | Pre | 1 | 0 | 1 | 1 | 0 | 0 | 0 | - | 2 | 0 | 1 | - | 2 | 0 | 0 | - | 0 | - | 0 | 1 | 1 | 0 | 0 | 0 |
| | 5.0 | Pre | 2 | 1 | 1 | 3 | 0 | 0 | 0 | - | 2 | 2 | 1 | - | 2 | 1 | 0 | - | 0 | - | 0 | 1 | 4 | 1 | 0 | 0 |
| | 5.0 | Post | 0 | 2 | 2 | 0 | 0 | 0 | 0 | 3 | 2 | 2 | 0 | 2 | 0 | 2 | 1 | 2 | 0 | 0 | 0 | 0 | 3 | 0 | 0 | 0 |
| 1 | 1.0 | Pre | 3 | 3 | 4 | 4 | 4 | 4 | 4 | 4 | 3 | 3 | - | 2 | 3 | 3 | 4 | - | 4 | 5 | 4 | 4 | 5 | 4 | 5 | 5 |
| | 5.0 | Pre | 3 | 4 | 4 | 4 | 4 | 4 | 4 | 4 | 4 | 4 | 4 | 4 | 4 | 4 | 4 | - | 5 | 4 | 4 | 4 | 5 | 4 | 4 | 5 |
| | 5.0 | Post | 3 | 3 | 3 | 2 | 3 | 2 | 0 | 4 | 4 | 4 | 2 | 2 | 4 | 4 | 4 | 3 | 0 | 4 | 0 | 4 | 4 | 4 | 0 | 1 |
| 2 | 1.0 | Pre | 3 | 3 | 2 | 3 | 3 | 4 | 3 | 4 | 0 | 4 | - | 4 | 4 | 3 | 4 | - | 4 | 5 | 4 | 4 | 5 | 4 | 4 | 5 |
| | 5.0 | Pre | 3 | 3 | 4 | 4 | 5 | 4 | 5 | 4 | 3 | 4 | - | 2 | 4 | 4 | 4 | - | 5 | 5 | 4 | 4 | 5 | 4 | 4 | 4 |
| | 5.0 | Post | 3 | 2 | 3 | 2 | 2 | 3 | 3 | 4 | 1 | 4 | 2 | 3 | 4 | 4 | 4 | 4 | 0 | 4 | 3 | 4 | 4 | 4 | 1 | 2 |
| 3 | 1.0 | Pre | 1 | 1 | 3 | 2 | 2 | 4 | 3 | - | 2 | 3 | 0 | - | 3 | 0 | 3 | - | 4 | - | 2 | 1 | 4 | 2 | 0 | 3 |
| | 5.0 | Pre | 3 | 2 | 4 | 4 | 3 | 4 | 4 | - | 3 | 4 | 2 | - | 4 | 0 | 3 | - | 4 | - | 4 | 4 | 4 | 3 | 4 | 5 |
| | 5.0 | Post | 0 | 1 | 2 | 2 | 0 | 1 | 0 | 2 | 3 | 2 | 0 | 0 | 1 | 2 | 3 | 1 | 0 | 0 | 0 | 0 | 0 | 0 | 0 | 0 |
| 5 | 5.0 | Pre | 0 | 0 | 3 | 1 | 2 | 0 | 0 | 0 | 2 | 0 | - | 0 | 1 | 0 | 0 | - | 0 | - | 0 | 0 | 1 | 0 | 0 | - |
| | 5.0 | Post | 0 | 0 | 0 | 0 | 0 | 0 | 0 | 1 | 0 | 0 | 0 | 2 | 1 | 0 | 0 | 0 | 0 | 1 | 0 | 0 | 0 | 0 | 0 | 0 |

A indicates Rate of Application in Kg per hectare

B indicates Pre- or Post-emergence Application

TABLE III CONTINUED...

| COMPOUND NO | A | B | TEST PLANTS | | | | | | | | | | | | | | | | | | | | | | |
|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv. | Ot | Dg | Pu | St | Ec | Sh | Ag | Cn |
| 6 | 1.0 | Pre | 4 | 3 | 3 | 2 | 4 | 4 | 4 | 4 | 1 | 4 | 3 | 4 | 3 | - | 3 | - | 4 | 4 | 4 | 4 | 5 | 3 | 2 | 2 |
| | 1.0 | Post | 3 | 2 | 2 | 0 | 2 | 2 | 3 | 2 | 3 | 1 | 2 | 1 | 3 | 2 | 2 | 1 | 1 | 4 | 2 | 3 | 3 | 2 | 2 | 1 |
| | 5.0 | Pre | 4 | 4 | 4 | 4 | 4 | 4 | 5 | 4 | 3 | 4 | 4 | 4 | 4 | - | 4 | - | 4 | 5 | 4 | 4 | 5 | 4 | 3 | 3 |
| | 5.0 | Post | 2 | 2 | 2 | 2 | 2 | 3 | 3 | 2 | 4 | 2 | 3 | 2 | 4 | 4 | 3 | 3 | 2 | 4 | 3 | 4 | 3 | 3 | 2 | 3 |
| | 0.05 | Pre | 3 | 1 | 1 | 0 | 0 | 3 | 2 | - | 0 | 3 | - | 2 | 2 | 0 | 0 | - | 0 | 4 | 4 | 3 | 4 | 2 | 0 | 0 |
| | 0.10 | Pre | 4 | 2 | 1 | - | 1 | 4 | 3 | - | 0 | 3 | - | 2 | 3 | 1 | 1 | - | 1 | 4 | 4 | 3 | 4 | 3 | 4 | - |
| | 0.20 | Pre | 4 | 3 | 3 | 0 | 3 | 4 | 4 | - | 1 | 4 | - | 3 | 4 | 2 | 3 | - | 4 | 5 | 4 | 4 | 5 | 4 | 4 | 3 |
| 7 | 1.0 | Pre | 4 | 2 | 0 | 1 | 2 | 4 | 4 | 4 | 3 | 4 | 1 | 3 | 4 | - | 2 | - | 4 | 4 | 4 | 4 | 5 | 4 | 0 | 0 |
| | 5.0 | Pre | 4 | 3 | 1 | 3 | 3 | 4 | 5 | 4 | 3 | 4 | 3 | 3 | 4 | - | 3 | - | 4 | 4 | 4 | 4 | 5 | 4 | 2 | 0 |
| | 5.0 | Post | 3 | 2 | 3 | 2 | 3 | 4 | 2 | 2 | 3 | 3 | 3 | 2 | 3 | 4 | 4 | 2 | 3 | 4 | 3 | 3 | 4 | 3 | 2 | 2 |
| | 0.2 | Pre | 4 | 2 | 0 | 0 | 0 | 3 | 3 | - | 0 | 4 | - | 2 | 2 | 1 | 0 | - | 4 | 4 | 4 | 3 | 4 | 4 | 1 | 3 |
| | 0.4 | Pre | 4 | 3 | 1 | - | 2 | 4 | 4 | - | 3 | 4 | - | 3 | 2 | 2 | 2 | - | 4 | 5 | 4 | 4 | 5 | 4 | 5 | 5 |
| | 0.8 | Pre | 4 | 3 | 2 | 0 | 3 | 4 | 4 | - | 3 | 4 | - | 2 | 3 | 3 | 3 | - | 4 | 5 | 4 | 4 | 5 | 4 | 4 | 4 |
| 8 | 1.0 | Pre | 4 | 3 | 2 | 3 | 4 | 4 | 5 | 4 | 3 | 4 | 3 | 3 | 4 | - | 3 | - | 4 | 4 | 4 | 4 | 5 | 4 | 4 | 5 |
| | 5.0 | Pre | 4 | 3 | 3 | 4 | 4 | 4 | 5 | 4 | 3 | 4 | 3 | 4 | 4 | - | 4 | - | 4 | 4 | 4 | 4 | 5 | 4 | 5 | 5 |
| | 5.0 | Post | 3 | 2 | 3 | 3 | 3 | 4 | 3 | 2 | 3 | 2 | 3 | 2 | 3 | 3 | 4 | 0 | 3 | 4 | 3 | 3 | 4 | 4 | 2 | 3 |
| | 0.05 | Pre | 3 | 0 | 0 | 0 | 0 | 2 | 3 | - | 0 | 2 | - | 0 | 1 | 0 | 2 | - | 0 | 4 | 3 | 3 | 4 | 0 | 2 | 4 |

TABLE III CONTINUED...

| COMPOUND NO | A | B | TEST PLANTS | | | | | | | | | | | | | | | | | | | | | | | |
|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
| 8 | 0.1 | Pre | 4 | 0 | 2 | 2 | 2 | 3 | 3 | - | 2 | 3 | - | 1 | 2 | 1 | 1 | - | 2 | 4 | 4 | 3 | 4 | 2 | 2 | 3 |
| | 0.2 | Pre | 4 | 0 | 3 | 2 | 3 | 4 | 4 | - | 2 | 4 | - | 3 | 4 | 3 | 3 | - | 3 | 5 | 4 | 4 | 5 | 4 | 5 | 5 |
| 9 | 1.0 | Pre | 3 | 1 | 1 | 1 | 0 | 4 | 4 | 3 | 0 | 4 | 1 | 3 | 3 | 0 | 2 | - | 3 | 4 | 4 | 4 | 4 | 3 | 1 | 0 |
| | 5.0 | Pre | 3 | 3 | 1 | 2 | 2 | 4 | 5 | 4 | 0 | 4 | 3 | 3 | 4 | 3 | 3 | - | 4 | 4 | 4 | 4 | 5 | 4 | 2 | 3 |
| 10 | 1.0 | Pre | 3 | 3 | 3 | 4 | 4 | 4 | 5 | 3 | 3 | 4 | - | 4 | 4 | - | 3 | - | 4 | 4 | 4 | 4 | 4 | 4 | 3 | 5 |
| | 5.0 | Pre | 3 | 3 | 4 | 4 | 4 | 4 | 5 | 4 | 3 | 4 | - | 4 | 4 | - | 3 | - | 5 | 4 | 4 | 4 | 5 | 4 | 4 | 5 |
| | 5.0 | Post | 2 | 2 | 3 | 4 | 3 | 3 | 3 | 4 | 4 | 3 | 3 | 4 | 4 | 4 | 3 | 2 | 2 | 3 | 3 | 4 | 4 | 2 | 2 | 3 |
| 11 | 1.0 | Pre | 4 | 4 | 4 | 4 | 4 | 5 | 5 | 4 | 4 | 4 | 4 | 4 | 4 | 4 | 4 | - | 5 | 4 | 5 | 4 | 5 | 4 | 5 | 5 |
| | 5.0 | Pre | 4 | 4 | 4 | 4 | 5 | 5 | 5 | 4 | 4 | 4 | 4 | 4 | 4 | 5 | 4 | - | 5 | 4 | 5 | 4 | 5 | 4 | 5 | 5 |
| | 5.0 | Post | 2 | 2 | 3 | 4 | 2 | 3 | 3 | 4 | 4 | 2 | 3 | 3 | 2 | 4 | 2 | 2 | 2 | 4 | 3 | 3 | 3 | 3 | 1 | 3 |
| | 0.1 | Pre | 3 | 3 | 2 | 2 | 2 | 4 | 3 | 3 | 1 | 4 | - | 2 | 3 | - | 2 | - | 3 | 4 | 4 | 4 | 4 | 0 | 1 | 0 |
| | 0.5 | Pre | 4 | 3 | 3 | 4 | 4 | 4 | 4 | 4 | 3 | 4 | - | 3 | 4 | - | 3 | - | 4 | 5 | 4 | 4 | 5 | 4 | 4 | 3 |
| | 0.05 | Pre | 4 | 3 | 1 | 1 | 0 | 4 | 2 | - | 0 | 2 | - | 2 | 3 | 2 | 1 | - | 0 | 5 | 4 | 4 | 4 | 2 | 3 | 2 |
| 12 | 1.0 | Pre | 4 | 4 | 4 | 4 | 4 | 4 | 5 | 4 | 3 | - | - | 4 | 4 | - | 4 | - | 4 | 5 | 5 | 4 | 5 | 4 | 4 | 4 |
| | 5.0 | Pre | 4 | 4 | 4 | 4 | 4 | 5 | 5 | 4 | 3 | - | - | 4 | 4 | - | 4 | - | 5 | 5 | 5 | 5 | 5 | 4 | 4 | 4 |

TABLE III CONTINUED...

| COMPOUND NO | A | B | TEST PLANTS | | | | | | | | | | | | | | | | | | | | | | | |
|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
| 12 | 5.0 | Post | 3 | 3 | 4 | 3 | 3 | 4 | 3 | 4 | 4 | 4 | 4 | 4 | 4 | - | 2 | 3 | 3 | 4 | 2 | 4 | 4 | 4 | 4 | 3 |
| | 0.05 | Post | 3 | 2 | 2 | 0 | 1 | 3 | 3 | - | - | 2 | - | 2 | 2 | 0 | 0 | - | 1 | 4 | 4 | 3 | 4 | 1 | 2 | 3 |
| 13 | 0.1 | Pre | 2 | 1 | 1 | 1 | 0 | 3 | 3 | 3 | - | 2 | - | - | 1 | 0 | 0 | - | 1 | 4 | 3 | 3 | 4 | 0 | 0 | - |
| | 0.2 | Pre | 3 | 2 | 0 | 0 | 0 | 4 | 4 | 3 | 0 | 2 | - | - | 2 | 0 | 0 | - | 3 | 4 | 4 | 4 | 4 | 3 | 2 | 0 |
| | 0.4 | Pre | 3 | 3 | 2 | 1 | 1 | 4 | 4 | 3 | 2 | 2 | - | - | 3 | 1 | 2 | - | 4 | 4 | 4 | 4 | 4 | 4 | 2 | - |
| | 1.0 | Post | 2 | 1 | 1 | 3 | 3 | 4 | 3 | 2 | 2 | 4 | 2 | 4 | 4 | 4 | 4 | 2 | 2 | 5 | 3 | 4 | 4 | 4 | 1 | 1 |

Names of test plants in Table III

| | |
|---|---|
| Sb | Sugar beet |
| Rp | Rape |
| Ct | Cotton |
| Sy | Soya bean |
| Mz | Maize |
| Ww | Winter wheat |
| Rc | Rice |
| Sn | Senecio vulgaris |
| Ip | Ipomoea purpurea |
| Am | Amaranthus retroflexus |
| Pi | Polygonum aviculare |
| Ca | Chenopodium album |
| Po | Portulaca oleracea |
| Ab | Abutilon theophrastii |
| Xs | Xanthium spinosa |
| Cv | Convolvulus arvensis |
| Ot | Cultivated oats and wild oats. The former are used in the pre-emergence test and the latter in the post-emergence test. |
| Dg | Digitaria sanguinalis |
| Pu | Poa annua |
| St | Setaria viridis |
| Ec | Echinochloa crus-galli |
| Sh | Sorghum halepense |
| Ag | Agropyron repens |
| Cn | Cyperus rotundus |

JED/vmc
9 Feb 79

1. Herbicidal compounds having the formula (I):-

$$R^1\underset{\underset{A}{\|}}{C}(H)C=C(X)R^2$$

(I)

wherein A is an atom of oxygen or sulphur; X is a 1-(1,2,4-triazolyl) radical or a 1-imidazolyl radical; and $R^1$ is an aliphatic or cycloaliphatic radical substituted by one or more of the following substituents: fluorine; chlorine; bromine; iodine; alkoxy; hydroxy; alkoxycarbonyl; acyloxy; cyano; carboxamido; nitro; mono- or di-alkylcarbamoyl; acylamido; mono- or dialkyl amino; phenyl; or mono- or dialkylsulphonamido; and $R^2$ is a phenyl or naphthyl radical optionally bearing one or more of the following substituents: fluorine; chlorine; bromine; iodine; cyano; nitro; alkyl of 1 to 6 carbon atoms optionally substituted by one or more alkoxy radicals or fluorine, chlorine, bromine, or iodine atoms; hydroxy; alkoxy of 1 to 6 carbon atoms optionally substituted by one or more phenyl radicals or alkoxy radicals of 1 to 6 carbon atoms; alkenyloxy or alkynyloxy of up to 6 carbon atoms; methylenedioxy or ethylenedioxy radicals optionally bearing one or more alkyl substituents in the methylene or ethylene moiety; alkylthio of 1 to 6 carbon atoms; phenyl; phenoxy; amino; mono- or di- alkylamino; acylamino; carboxy; carbamoyl; mono- or di- alkylcarbamoyl; sulphamoyl; or mono-or di- alkylsulphamoyl; or an acid addition salt or metal complex thereof.

2. Herbicidal compounds as claimed in claim 1, wherein the group $R^1$ is a group of formula:

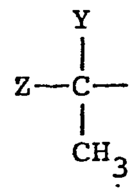

wherein Y is a methyl group or a 2-cyanoethyl group, and Z is a hydroxy group; an alkoxy group of 1 to 6 carbon atoms; an alkanoyloxy group of 2 to 6 carbon atoms; a benzoyloxy group optionally substituted by one or more methyl groups or atoms of fluorine, chlorine, bromine or iodine; or a 2-cyanoethyl group.

3. Herbicidal compounds as claimed in claim 1 or claim 2, wherein the group $R^2$ is a phenyl radical optionally substituted by one or more alkyl groups of 1 to 4 carbon atoms, alkoxy groups of 1 to 6 carbon atoms, or fluorine, chlorine, bromine or iodine atoms.

4. Herbicidal compounds as claimed in any of claims 1 to 3 wherein the group $R^2$ is a 2-alkoxy-5-halogenophenyl group.

5. A process of inhibiting the growth of unwanted plants, which comprises applying to the plants, or to the locus of the plants, a herbicidally effective amount of a compound of the formula (I) defined in claim 1.

6. A process as claimed in claim 5 wherein the rate of application of the compound is from 0.01 to 5.0 kilograms per hectare.

7. Herbicidal compositions comprising as an active ingredient a compound of the formula (I) defined in claim 1, in admixture with a carrier comprising a solid or liquid diluent.

8. Herbicidal compositions comprising a compound of the formula (I) defined in claim 1 in admixture with an additional herbicide other than a compound of the formula (I).

9. A process of preparing compounds of the formula (I) defined in claim 1, which comprises reacting a compound of the formula:

$R^1$—CCH(Hal)CH(Hal)$R^2$
‖
A

wherein Hal stands for chlorine or bromine, with the anion of 1,2,4-triazole or imidazole in an aprotic solvent at a elevated temperature, and recovering the compound of formula (I) by conventional methods.